# EUROPEAN PATENT APPLICATION

(11) **EP 4 406 947 A1**
(43) Date of publication of application: **31.07.2024**
(21) Application number: 22872872.1
(22) Date of filing: 20.09.2022
(51) Int. Cl.: C07D 401/10, C07D 401/14, C07D 409/10, C07D 409/14, C07D 413/10, C07D 413/14, C07D 417/10, C07D 417/14, C09K 11/06, H05B 33/02, H10K 50/00, H10K 50/16, H10K 50/15

(54) **COMPOUND AND ORGANIC ELECTROLUMINESCENT ELEMENT**

(30) Priority: 21.09.2021 JP 2021152958
(71) Applicant: Hodogaya Chemical Co., Ltd., Tokyo, 105-0021 (JP)
(72) Inventor: KASE, Kouki, Tokyo 105-0021 (JP); CHIBA, Eriko, Tokyo 105-0021 (JP); YANG, Byung-Sun, Tokyo 105-0021 (JP); HWANG, Moon-Chan, Tokyo 105-0021 (JP); HIRAYAMA, Yuta, Tokyo 105-0021 (JP); HAYASHI, Shuichi, Tokyo 105-0021 (JP)
(74) Representative: Forstmeyer, Dietmar
(86) International application number: PCT/JP2022/034920
(87) International publication number: WO 2023/048118

(57) **Abstract**

It is an object of the present invention to provide a compound that has a high refractive index and a low extinction coefficient in a wavelength range of 450 to 750 nm, which is favorably used as a material for a capping layer of an organic EL element. Also, it is another object of the present invention to provide an organic EL element whose light extraction efficiency has been improved by using the compound. The present invention is directed to a compound represented by a general formula (1) or (2) below: 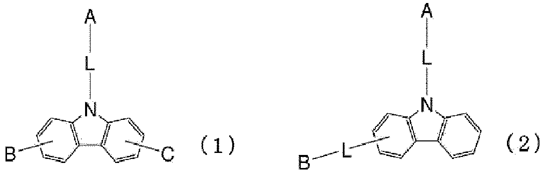 where A represents a substituted or unsubstituted aromatic hydrocarbon group or the like, B and C are optionally the same or different, and represent an unsubstituted naphthyl group, an unsubstituted quinolyl group, or the like, and each L is optionally the same or different, and represents a single bond, a substituted or unsubstituted divalent aromatic hydrocarbon group, or the like.

## Description

### Technical Field

The present invention relates to a compound suitable for a self-luminescent electronic element favorably used in various display devices, particularly relates to a compound suitable for an organic electroluminescent element (hereinafter abbreviated as an "organic EL element") and an organic EL element, an electronic device, or an electronic element using the compound.

### Background Art

Since organic EL elements are self-luminescent elements, they are brighter, have superior display viewability, and can provide a clearer display, compared with liquid crystal elements. For these reasons, active studies have been carried out on organic EL elements.

In 1987, C. W. Tang et al. of Eastman Kodak Company produced a practical organic EL element made of an organic material, by developing an element having a stacked layer structure in which various functions were assigned to different materials. They achieved a high luminance of 1,000 cd/m² or higher at a voltage of 10 V or less by stacking a layer of a fluorescent body capable of transporting electrons and a layer of an organic substance capable of transporting holes, injecting both charges into the fluorescent body layer, and thereby causing the layer to emit light (see Patent Literatures 1 and 2, for example).

Many improvements have been heretofore made to organic EL elements to put them to practical use, and among electroluminescent elements in which various functions of the stacked layer structure are subdivided even further, and an anode, a hole injection layer, a hole transport layer, a light emitting layer, an electron transport layer, an electron injection layer, and a cathode are sequentially provided on a substrate, light emitting elements having a bottom-emission structure that emits light from a bottom portion are achieving high efficiency and durability (see Non-Patent Literature 1, for example).

Recently, light emitting elements with a top emission structure in which a metal having a high work function is used for an anode and light is emitted from the top are coming into use. Although light emitting elements with a bottom emission structure in which light is extracted from the bottom having a pixel circuit have an issue in that the area of the light emitting portion is limited, light emitting elements with a top emission structure are advantageous in that a large light emitting portion can be realized because light extracted from the top is not blocked by the pixel circuit. In light emitting elements with a top emission structure, translucent electrodes made of LiF/Al/Ag (see Non-Patent Literature 2, for example), Ca/Mg (see Non-Patent Literature 3, for example), LiF/MgAg, or the like are used for a cathode.

In such light emitting elements, when light that is emitted by a light emitting layer and incident on another film is incident at a certain angle or more, the light is totally reflected at an interface between the light emitting layer and the other film. Thus, light that can be used has been limited to only a part of the emitted light. Recently, light emitting elements have been proposed in which a "capping layer" with a high refractive index is provided on the outside of a translucent electrode with a low refractive index, in order to improve the light extraction efficiency (see Non-Patent Literatures 2 and 3, for example).

Regarding the effect of the capping layer in light emitting elements with a top emission structure, while a light emitting element using Ir(ppy)₃ as a light emitting material has a current efficiency of 38 cd/A in the case of not having a capping layer, the light emitting element has a current efficiency of 64 cd/A in the case of using a ZnSe film with a thickness of 60 nm as a capping layer, which indicates that the efficiency is improved about 1.7 times. Furthermore, it is indicated that a maximum point of the transmittance and a maximum point of the efficiency of the translucent electrode and the capping layer do not absolutely match each other, and the maximum point of the light extraction efficiency is determined by interference effects (see Non-Patent Literature 3, for example).

Conventionally, it has been proposed to use a fine metal mask to form a capping layer, but this configuration is problematic in that a metal mask is deformed by heat when used at a high temperature, which deteriorates the positioning precision. For example, ZnSe has a high melting point of 1100°C or higher (see Non-Patent Literature 3, for example), and thus vapor deposition cannot be performed at an accurate position with a fine metal mask, which may affect the light emitting element itself. Furthermore, film forming through sputtering also affects the light emitting element, and thus a capping layer using an inorganic material as a constituent material cannot be suitably used.

In addition, in the case in which tris(8-hydroxyquinoline)aluminum (hereinafter abbreviated as "Alq₃") is used for a capping layer for adjusting the refractive index (see Non-Patent Literature 2, for example), Alq₃, which is known as an organic EL material that is commonly used as a green light emitting material or an electron transport material, is poor in absorption at around 450 nm, which is used for a blue light emitting material, and thus, when it is used for blue light emitting elements, there is a problem in that both the color purity and the light extraction efficiency deteriorate.

In order to improve the element characteristics of an organic EL element and significantly improve the light extraction efficiency thereof, there is a demand for a material for a capping layer that has a high refractive index and a low extinction coefficient, is stable in a thin film state, and has excellent durability.

### Citation List

### Patent Literatures

Patent Literature 1: US 5792557
Patent Literature 2: US 5639914
Patent Literature 3: WO 2014/009310
Patent Literature 4: US 2014/0225100A1

### Non-Patent Literatures

Non-Patent Literature 1: Proceedings of the 9th Meeting of the Japan Society of Applied Physics, pp. 55-61 (2001)
Non-Patent Literature 2: Appl. Phys. Let., 78, 544 (2001)
Non-Patent Literature 3: Appl. Phys. Let., 82, 466 (2003)
Non-Patent Literature 4: Chem. Rev. 2016, 116, 12564
Non-Patent Literature 5: Angew. Chem. 2003, 42, 5400
Non-Patent Literature 6: Appl. Phys. Let., 98, 083302 (2011)

### Summary of Invention

### Technical Problem

It is an object of the present invention to provide a compound that has a high refractive index and a low extinction coefficient in a wavelength range of 450 to 750 nm, which is favorably used as a material for a capping layer of an organic EL element. Also, it is another object of the present invention to provide an organic EL element whose light extraction efficiency has been improved by using the compound.

A compound suitable for a capping layer of an organic EL element is required to have the following physical properties: (1) a high refractive index, (2) a low extinction coefficient, (3) vapor-depositability, (4) good stability in a thin film state, and (5) a high glass transition point. Also, an organic EL element that is to be provided by the present invention is required to have the following physical properties: (1) high light extraction efficiency, (2) no deterioration in the color purity, (3) light transmittance without change over time, and (4) a long life.

### Solution to Problem

To achieve the above-described objects, the inventors of the present invention optimized the molecular design focusing on the fact that compounds with a carbazole skeleton have excellent stability in a thin film state and durability, and thus developed a material that has a high refractive index and a low extinction coefficient in a wavelength range of 450 to 750 nm. Furthermore, the inventors produced an organic EL element using the compound and thoroughly evaluated the properties thereof, as a result of which it was found that the conventional problems are solved, and the present invention was accomplished.

That is to say, the present invention is directed to a compound represented by a general formula (1) or (2) below and an organic EL element using the same.

1) A compound represented by a general formula (1) or (2) below. 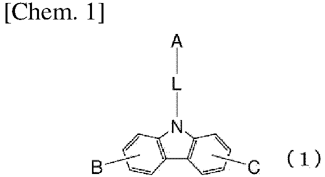 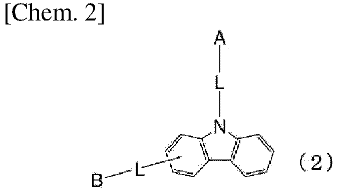
   In the formulas, A represents a substituted or unsubstituted aromatic hydrocarbon group, a substituted or unsubstituted aromatic heterocyclic group, or a substituted or unsubstituted fused polycyclic aromatic group,
   B and C are optionally the same or different, and represent an unsubstituted naphthyl group, an unsubstituted quinolyl group, an unsubstituted benzofuranyl group, or an unsubstituted benzothienyl group, and
   Each L is optionally the same or different, and represents a single bond, a substituted or unsubstituted divalent aromatic hydrocarbon group, a substituted or unsubstituted divalent aromatic heterocyclic group, or a substituted or unsubstituted divalent fused polycyclic aromatic group.
2) The compound according to 1), wherein the compounds represented by the general formulas (1) and (2) mentioned above are respectively the compounds represented by general formulas (3) and (4) below. 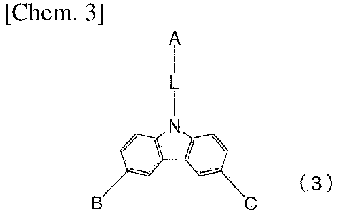 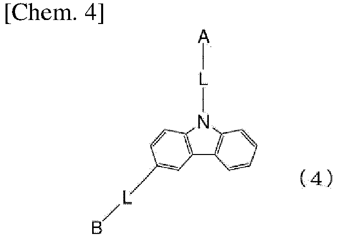 In the formulas, A, B, C, and L are the same as those defined in the general formulas (1) and (2) above.
3) The compound according to 2), wherein B and C in the general formulas (3) and (4) mentioned above are optionally the same or different, and represent a 2-naphthyl group, a 3-quinolyl group, a 2-benzofuranyl group, or a 2-benzothienyl group.
4) The compound according to 3), wherein each L in the general formulas (3) and (4) mentioned above is optionally the same or different, and represents a single bond, a substituted or unsubstituted phenylene group, a substituted or unsubstituted biphenylene group, or a substituted or unsubstituted naphthylene group.
5) An organic EL element at least including an anode electrode, a hole transport layer, a light emitting layer, an electron transport layer, a cathode electrode, and a capping layer arranged in this order, wherein the capping layer contains the compound according to any one of 1) to 4).
6) The organic EL element according to 5), wherein the refractive index of the capping layer in a wavelength range of 450 to 750 nm is 1.70 or more.
7) The organic EL element as set forth in 5), wherein the capping layer is stacked layers respectively constituted by two or more types of compounds or a mixed layer containing two or more types of compounds, and at least one of the two or more types of compounds is the compound according to any one of 1) to 4).
8) An electronic element including a pair of electrodes and an organic layer held therebetween, wherein the organic layer contains the compound according to any one of 1) to 4).
9) An electronic device comprising the electronic element according to 8).

The "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by A in the general formulas (1), (2), (3), and (4) may specifically refer to a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzooxazolyl group, a benzothiazolyl group, a quinoxalinyl group, a quinazolinyl group, a benzimidazolyl group, a pyrazolyl group, a dibenzofuranyl group, a dibenzothienyl group, a naphthyridinyl group, a phenanthrolinyl group, an acridinyl group, a carbolinyl group, or the like, and is preferably selected from aryl groups having 6 to 30 carbon atoms and heteroaryl groups having 2 to 20 carbon atoms.

The "divalent aromatic hydrocarbon group", the "divalent aromatic heterocyclic group", or the "divalent fused polycyclic aromatic group" in the "substituted or unsubstituted divalent aromatic hydrocarbon group", the "substituted or unsubstituted divalent aromatic heterocyclic group", or the "substituted or unsubstituted divalent fused polycyclic aromatic group" represented by L in the general formulas (1), (2), (3), and (4) may refer to a divalent group obtained by reducing one hydrogen atom from the groups given as an example of the "aromatic hydrocarbon group", the "aromatic heterocyclic group", or the "fused polycyclic aromatic group" represented by A in the general formulas (1), (2), (3), and (4).

The "substituent" in the "substituted aromatic hydrocarbon group", the "substituted aromatic heterocyclic group", or the "substituted fused polycyclic aromatic group" represented by A and L in the general formulas (1), (2), (3), and (4) may specifically refer to: a heavy hydrogen atom, a cyano group, or a nitro group; a halogen atom such as a fluorine atom, a chlorine atom, a bromine atom, or an iodine atom; a silyl group such as a trimethylsilyl group or a triphenylsilyl group; a linear or branched alkyl group having 1 to 6 carbon atoms such as a methyl group, an ethyl group, or a propyl group; a linear or branched alkyloxy group having 1 to 6 carbon atoms such as a methyloxy group, an ethyloxy group, or a propyloxy group; an alkenyl group such as a vinyl group or an aryl group; an aryloxy group such as a phenyloxy group or a tolyloxy group; an arylalkyloxy group such as a benzyloxy group or a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, or a triphenylenyl group; a pyridyl group, a thienyl group, a furyl group, a pyrrolyl group, a quinolyl group, an isoquinolyl group, a benzofuranyl group, a benzothienyl group, an indolyl group, a carbazolyl group, a benzooxazolyl group, a benzothiazolyl group, a quinoxalinyl group, a quinazolinyl group, a benzimidazolyl group, a pyrazolyl group, a dibenzofuranyl group, a dibenzothienyl group, a carbolinyl group, or the like, and may further refer to an aryl group having 6 to 30 carbon atoms or a heteroaryl group having 2 to 20 carbon atoms. These substituents may be further substituted with the above-listed substituents. Furthermore, adjacent benzene rings substituted with these substituents or a plurality of adjacent substituents on the same benzene ring may be bonded to each other via a single bond, a substituted or unsubstituted methylene group, an oxygen atom, or a sulfur atom to form a ring.

In the present invention, A in the general formulas (1) to (4) is preferably a substituted or unsubstituted phenyl group, a substituted or unsubstituted naphthyl group, a substituted or unsubstituted phenanthrenyl group, a substituted or unsubstituted quinolyl group, a substituted or unsubstituted benzofuranyl group, a substituted or unsubstituted benzothienyl group, a substituted or unsubstituted dibenzofuranyl group, a substituted or unsubstituted dibenzothienyl group, a substituted or unsubstituted benzooxazolyl group, or a substituted or unsubstituted benzothiazolyl group.

In the present invention, L in the general formulas (1) to (4) is preferably a single bond, a substituted or unsubstituted phenylene group, a substituted or unsubstituted biphenylene group, or a substituted or unsubstituted naphthylene group, and more preferably a single bond, a substituted or unsubstituted 1,4-phenylene group, a substituted or unsubstituted 1,3-phenylene group, a substituted or unsubstituted 4,4'-biphenylene group, a substituted or unsubstituted 3,4'-biphenylene group, or a substituted or unsubstituted 2,7-naphthylene group.

In the present invention, B and C in the general formulas (1) to (4) are optionally the same or different, and are preferably a substituted or unsubstituted naphthyl group, a substituted or unsubstituted quinolyl group, or a substituted or unsubstituted benzofuranyl group.

B and C in the general formulas (1) and (3) are optionally the same or different, and are preferably a 2-naphthyl group, a 3-quinolyl group, a 2-benzofuranyl group, or a 2-benzothienyl group, and more preferably an unsubstituted 2-naphthyl group or an unsubstituted 3-quinolyl group. Furthermore, B and C are preferably the same.

B in the general formulas (2) and (4) is preferably a 2-naphthyl group, a 3-quinolyl group, a 2-benzofuranyl group, or a 2-benzothienyl group, and more preferably an unsubstituted 3-quinolyl group or an unsubstituted 2-benzofuranyl group.

In the present invention, the compound represented by the general formula (1) is preferably the compound represented by the general formula (3), and the compound represented by the general formula (2) is preferably the compound represented by the general formula (4).

In the organic EL element of the present invention, the capping layer has a thickness of preferably 30 to 120 nm, and more preferably 40 to 80 nm.

### Advantageous Effects of Invention

Since the compounds represented by the general formulas (1) and (2) above of the present invention have (1) a high refractive index in a wavelength range of 450 to 750 nm, (2) a low extinction coefficient, (3) vapor-depositability, (4) good stability in a thin film state, and (5) a high thermal resistance, it is possible to obtain an organic EL element whose light extraction efficiency has been significantly improved, by providing a capping layer using such a compound, outside the transparent or translucent electrode of the organic EL element.

### Brief Description of Drawings

[Fig. 1] The structures of Compounds (1-1) to (1-12) as examples of the compound of the present invention.
[Fig. 2] The structures of Compounds (1-13) to (1-24) as examples of the compound of the present invention.
[Fig. 3] The structures of Compounds (1-25) to (1-36) as examples of the compound of the present invention.
[Fig. 4] The structures of Compounds (1-37) to (1-48) as examples of the compound of the present invention.
[Fig. 5] The structures of Compounds (1-49) to (1-57) as examples of the compound of the present invention.
[Fig. 6] The structures of Compounds (1-58) to (1-66) as examples of the compound of the present invention.
[Fig. 7] The structures of Compounds (1-67) to (1-75) as examples of the compound of the present invention.
[Fig. 8] The structures of Compounds (1-76) to (1-83) as examples of the compound of the present invention.
[Fig. 9] The structures of Compounds (1-84) to (1-92) as examples of the compound of the present invention.
[Fig. 10]The structures of Compounds (1-93) to (1-94) as examples of the compound of the present invention.
[Fig. 11] An example of the configuration of the organic EL element of the present invention.

### Description of Embodiment

The carbazole compound represented by the general formulas (1) and (2) above is a novel compound, but can be synthesized, for example, according to known coupling reactions using a copper catalyst, a palladium catalyst, or the like (see Non-Patent Literatures 4 and 5, for example).

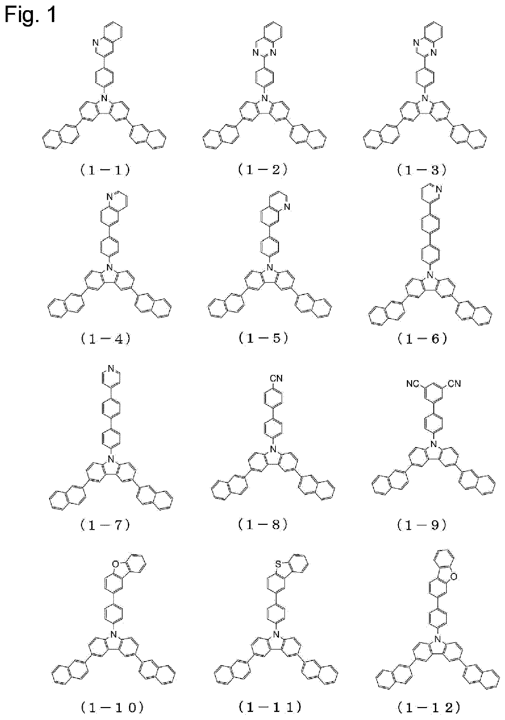

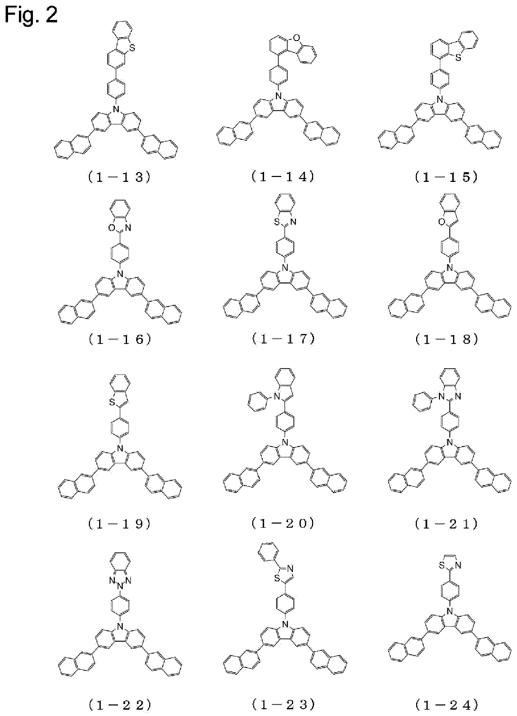

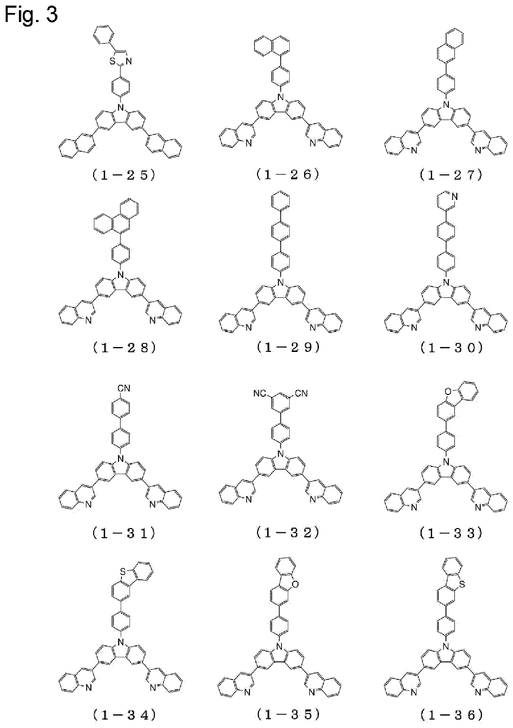

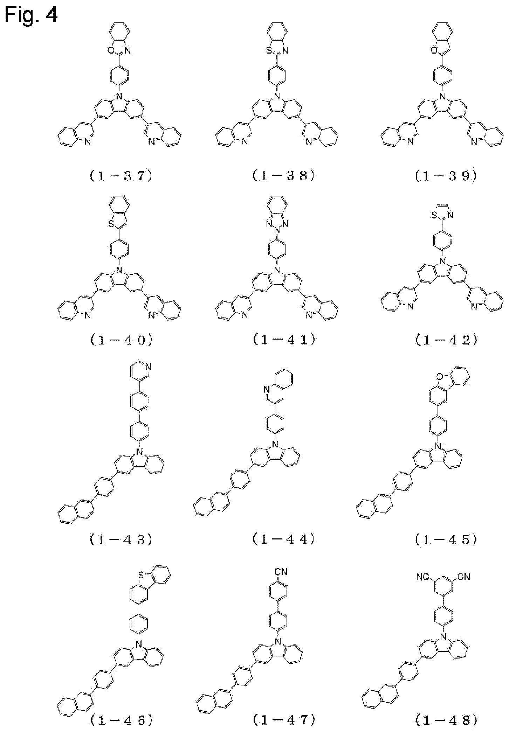

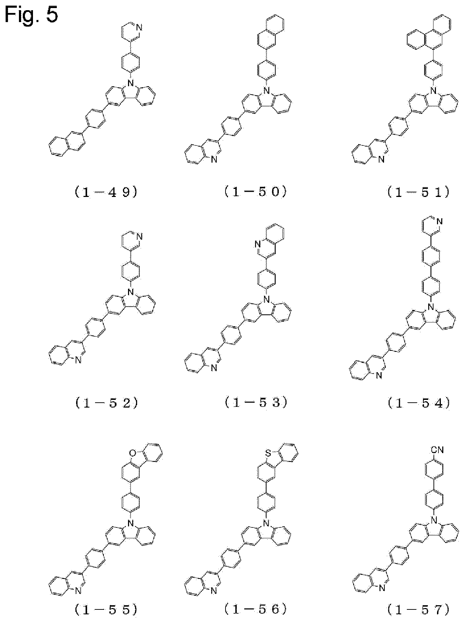

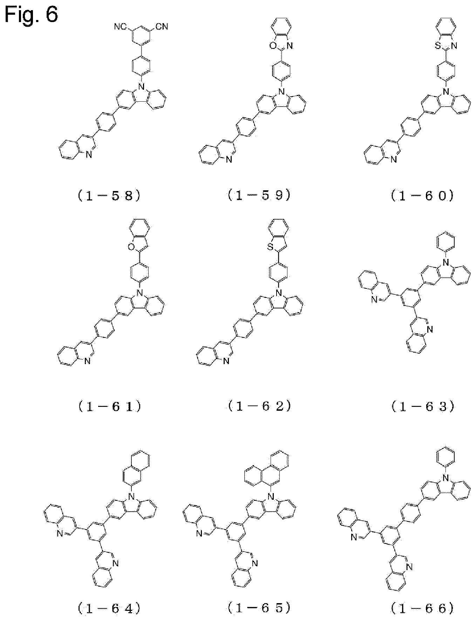

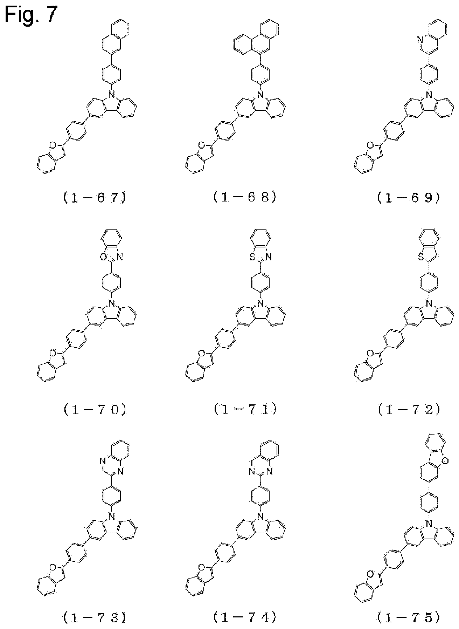

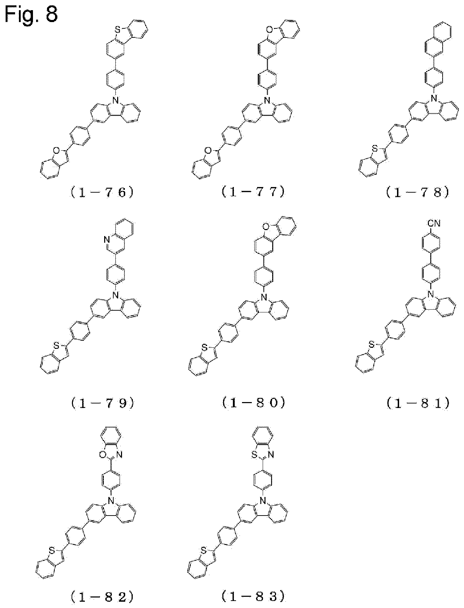

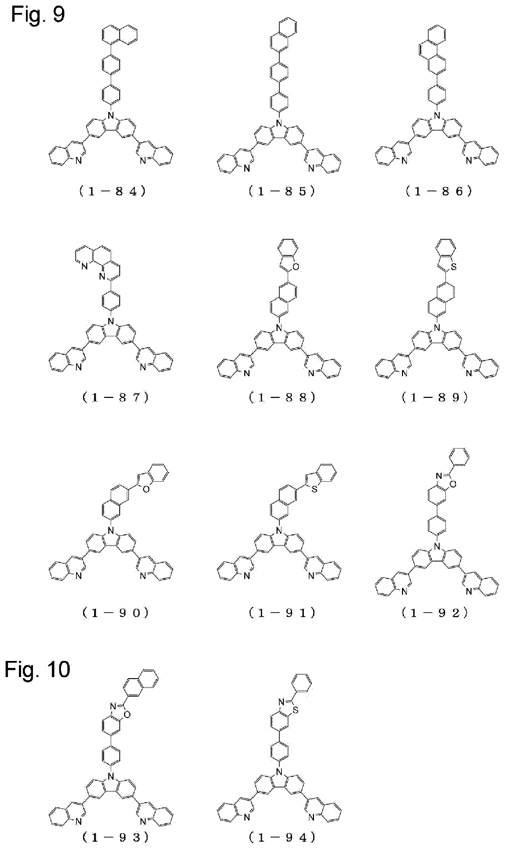

Figs. 1 to 10 show specific examples of preferred compounds among those represented by the general formulas (1) and (2) above, but the invention is not limited to these compounds.

These is no particular limitation on the method for purifying the compounds represented by the general formulas (1) and (2) above, but examples thereof include known methods used for purification of organic compounds, such as purification by column chromatography, adsorption purification using silica gel, activated carbon, activated clay, or the like, recrystallization purification and crystallization purification using solvents, and sublimation purification. Compounds can be identified by NMR analysis. Furthermore, the melting point, the glass transition point (Tg), and the refractive index are preferably measured as physical properties. The melting point is an indicator of vapor deposition properties, the glass transition point (Tg) is an indicator of the stability in a thin film state, and the refractive index is an indicator of the improvement of light extraction efficiency.

The melting point and the glass transition point (Tg) can be obtained by performing measurement on a powder using a high-sensitivity differential scanning calorimeter (DSC3100SA manufactured by Bruker AXS K.K.).

The refractive index and the extinction coefficient can be obtained by performing measurement on an 80-nm thin film formed on a silicon substrate, using a spectroscopic measurement device (F10-RT-UV manufactured by Filmetrics).

The organic EL element for use as a light emitting element with a top emission structure may have a structure constituted by an anode, a hole transport layer, a light emitting layer, an electron transport layer, a cathode, and a capping layer sequentially provided on a glass substrate, a structure in which a hole injection layer is further provided between the anode and the hole transport layer, a structure in which an electron blocking layer is further provided between the hole transport layer and the light emitting layer, a structure in which a hole blocking layer is further provided between the light emitting layer and the electron transport layer, and a structure in which an electron injection layer is further provided between the electron transport layer and the cathode. In these multilayer structures, a single organic layer may have the functions of a plurality of layers, and, for example, it is possible to adopt a configuration in which an organic layer serves as both the hole injection layer and the hole transport layer, a configuration in which an organic layer serves as both the hole transport layer and the electron blocking layer, a configuration in which an organic layer serves as both the hole blocking layer and the electron transport layer, a configuration in which an organic layer serves as both the electron transport layer and the electron injection layer, and the like. Furthermore, two or more organic layers having the same function may be stacked, and, for example, it is possible to adopt a configuration in which two hole transport layers are stacked, a configuration in which two light emitting layers are stacked, a configuration in which two electron transport layers are stacked, a configuration in which two capping layers are stacked, and the like.

The total film thickness of the layers of the organic EL element is preferably approximately from 200 to 750 nm, and more preferably approximately from 350 to 600 nm. Furthermore, the film thickness of the capping layer is, for example, preferably from 30 to 120 nm, and more preferably from 40 to 80 nm. In this case, it is possible to obtain good light extraction efficiency. Note that the film thickness of the capping layer may be changed as appropriate according to the type of light emitting material used in the light emitting element, the thickness of the organic EL element excluding the capping layer, and the like.

An electrode material having a high work function, such as ITO or gold, is used for the anode of the organic EL element of the present invention.

Arylamine compounds having a molecular structure in which three or more triphenylamine structures are bonded to each other via a single bond or a divalent group not containing a hetero atom, for example, starburst triphenylamine derivatives, materials such as various triphenylamine tetramers, porphyrin compounds typified by copper phthalocyanine, acceptor type heterocyclic compounds such as hexacyanoazatriphenylene, and coating type polymer materials can be used for the hole injection layer of the organic EL element of the present invention. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using vapor deposition, as well as a known method such as spin coating, or inkjet printing.

Benzidine derivatives such as N,N'-diphenyl-N,N'-di(m-tolyl)benzidine (hereinafter abbreviated as "TPD"), N,N'-diphenyl-N,N'-di(α-naphthyl)benzidine, and N,N,N',N'-tetrabiphenylyl benzidine, 1,1-bis[4-(di-4-tolylamino)phenyl]cyclohexane, in particular, arylamine compounds having a molecular structure in which two triphenylamine structures are bonded to each other via a single bond or a divalent group not containing a hetero atom, for example, N,N,N',N'-tetrabiphenylyl benzidine and the like are preferably used for the hole transport layer of the organic EL element. Also, arylamine compounds having a molecular structure in which three or more triphenylamine structures are bonded to each other via a single bond or a divalent group not containing a hetero atom, for example, various triphenylamine trimers and tetramers are preferably used. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. Furthermore, coating type polymer materials such as poly(3,4-ethylenedioxythiophene)/poly(styrenesulfonate) can be used for the hole injection and transport layers. With these materials, a thin film can be formed using vapor deposition, as well as a known method such as spin coating, or inkjet printing.

Furthermore, a material that is obtained by p-doping a material commonly used for the hole injection layer and the hole transport layer with trisbromophenylamine hexachloroantimony, a radialene derivative (see Patent Literature 3, for example), or the like, a polymer compound that has the structure of a benzidine derivative, such as TPD, in a partial structure thereof, and the like can be used for the hole injection layer and the hole transport layer.

Furthermore, it is also possible to stack an electron blocking layer on the organic EL element. Compounds having an electron blocking effect, such as: carbazole derivatives such as 4,4',4"-tri(N-carbazolyl)triphenylamine (hereinafter abbreviated as "TCTA"), 9,9-bis[4-(carbazole-9-yl)phenyl]fluorene, 1,3-bis(carbazole-9-yl)benzene (hereinafter abbreviated as "mCP"), and 2,2-bis(4-carbazole-9-ylphenyl)adamantane; and compounds that have a triphenylsilyl group and a triarylamine structure and are typified by 9-[4-(carbazole-9-yl)phenyl]-9-[4-(triphenylsilyl)phenyl]-9H-fluorene, can be used for the electron blocking layer. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using vapor deposition, as well as a known method such as spin coating, or inkjet printing.

In addition to metal complexes of quinolinol derivatives such as Alq₃, various types of metal complexes, an anthracene derivative, a bisstyrylbenzene derivative, a pyrene derivative, an oxazole derivative, a poly(p-phenylene vinylene) derivative, and the like can be used for the light emitting layer of the organic EL element. Moreover, the light emitting layer may also be formed using a host material and a dopant material, and, as the host material, an anthracene derivative is preferably used, and heterocyclic compounds having an indole ring as a partial structure of a fused ring, heterocyclic compounds having a carbazole ring as a partial structure of a fused ring, a carbazole derivative, a thiazole derivative, a benzimidazole derivative, a polydialkylfluorene derivative, and the like can be used in addition to the above-listed light emitting materials. As the dopant material, quinacridone, coumarin, rubrene, perylene, and derivatives thereof; a benzopyran derivative; a rhodamine derivative; an aminostyryl derivative; and the like can be used, and green light emitting materials are preferably used. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked.

Furthermore, it is also possible to use a phosphorescent emitter as the light emitting material. As the phosphorescent emitter, a phosphorescent emitter of a metal complex of iridium, platinum, or the like can be used. Examples thereof include a green phosphorescent emitter such as Ir(ppy)₃, a blue phosphorescent emitter such as FIrpic or FIr6, a red phosphorescent emitter such as Btp₂Ir (acac), and the like, and a green phosphorescent emitter is preferably used. In this case, as host materials having hole injectability and transportability, carbazole derivatives such as 4,4'-di(N-carbazolyl)biphenyl, TCTA, and mCP can be used as the host material. Furthermore, as host materials having electron transportability, p-bis(triphenylsilyl)benzene, 2,2',2"-(1,3,5-phenylene)-tris(1-phenyl-1H-benzimidazole), and the like can be used to produce a high-performance organic EL element.

In order to avoid concentration quenching, it is preferable that doping of the host material with a phosphorescent light emitting material is performed within a range of 1 to 30 wt% with respect to the entire light emitting layer through co-deposition.

As the light emitting material, materials that emit delayed fluorescence, such as CDCB derivatives such as PIC-TRZ, CC2TA, PXZ-TRZ, and 4CzIPN, can also be used (see Non-Patent Literature 6, for example). With these materials, a thin film can be formed using vapor deposition, as well as a known method such as spin coating, or inkjet printing.

Furthermore, it is also possible to stack a hole blocking layer on the organic EL element. Compounds having a hole blocking effect, such as a phenanthroline derivative such as bathocuproine, a metal complex of a quinolinol derivative, such as aluminum(III)bis(2-methyl-8-quinolinato)-4-phenylphenolate (hereinafter abbreviated as BAlq), various types of rare-earth complexes, a triazole derivative, a triazine derivative, a pyrimidine derivative, an oxadiazole derivative, a benzoazole derivative, and the like can be used for the hole blocking layer. These materials may also serve as the material for the electron transport layer. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using vapor deposition, as well as a known method such as spin coating, or inkjet printing.

In addition to metal complexes of quinolinol derivatives such as Alq₃ and BAlq, various types of metal complexes, a triazole derivative, a triazine derivative, a pyrimidine derivative, an oxadiazole derivative, a pyridine derivative, a benzimidazole derivative, a benzoazole derivative, a thiadiazole derivative, an anthracene derivative, a carbodiimide derivative, a quinoxaline derivative, a pyridoindole derivative, a phenanthroline derivative, a silole derivative, and the like can be used for the electron transport layer of the organic EL element. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using vapor deposition, as well as a known method such as spin coating, or inkjet printing.

Alkali metal salts such as lithium fluoride and cesium fluoride, alkaline earth metal salts such as magnesium fluoride, metal complexes of quinolinol derivatives such as lithium quinolinol, metal oxides such as aluminum oxide, metals such as ytterbium (Yb), samarium (Sm), calcium (Ca), strontium (Sr), and cesium (Cs), and the like can be used for the electron injection layer of the organic EL element. When an electron transport layer and a cathode are suitably selected, the electron injection layer can be omitted.

Furthermore, a material obtained by n-doping a material commonly used for the electron injection layer and the electron transport layer with a metal such as cesium can be used for the electron injection layer or the electron transport layer.

An electrode material having a low work function such as aluminum, an alloy having an even lower work function such as a magnesium-silver alloy, a magnesium-calcium alloy, a magnesium-indium alloy, or an aluminum-magnesium alloy, ITO, IZO, or the like is used as the electrode material of the cathode of the organic EL element.

The compounds represented by the general formulas (1) and (2) above are used for the capping layer of the organic EL element of the present invention. These materials may be used alone to form a layer, or may be used as a mixture with another material to form a single layer. Furthermore, it is also possible to adopt a structure in which layers that are each formed using one of the above-listed materials alone are stacked; a structure in which layers that are each formed using a mixture of any of the above-listed materials with another material are stacked; or a structure in which a layer that is formed using one of the above-listed materials alone and a layer that is formed using a mixture of any of the above-listed materials with another material are stacked. With these materials, a thin film can be formed using vapor deposition, as well as a known method such as spin coating, or inkjet printing.

The compounds represented by the general formulas (1) and (2) above have a refractive index in a wavelength range of 450 to 700 nm of preferably 1.70 or more, and more preferably 1.85 or more. That is to say, the capping layer has a refractive index in a wavelength range of 450 to 750 nm of preferably 1.70 or more, and more preferably 1.85 or more.

In the description above, an organic EL element with a top emission structure has been described, but the present invention is not limited thereto, and can also be applied in a similar manner to an organic EL element with a bottom emission structure or an organic EL element with a dual emission structure in which light is emitted from both of the top and the bottom. In these cases, an electrode located in a direction in which light is extracted from the light emitting element to the outside is preferably transparent or translucent.

Hereinafter, an embodiment of the present invention will be described in greater detail using examples. However, the present invention is not limited to the examples below as long as it is within the gist thereof.

### Examples

### Example 1

### <Synthesis of 3,6-di-naphthalene-2-yl-9-(4-quinoline-3-yl-phenyl)-9H-carbazole (Compound 1-1)>

In this example, 50.0 g of 3,6-dibromo-9H-carbazole, 58.2 g of 2-naphthaleneboronic acid, 7.1 g of tetrakis(triphenylphosphine)palladium(0), and 85.1 g of potassium carbonate were added to a reaction vessel, and the mixture was refluxed and stirred in toluene/EtOH/H₂O mixed solvent overnight. After the mixture was allowed to cool, ethyl acetate/H₂O was added to the system, and the organic layer was extracted through extraction and separation and concentrated to obtain a crude product. The obtained crude product was purified through crystallization using monochlorobenzene/n-heptane mixed solvent to obtain 35.9 g of 3,6-di-naphthalene-2-yl-9H-carbazole (yield: 55.6%).

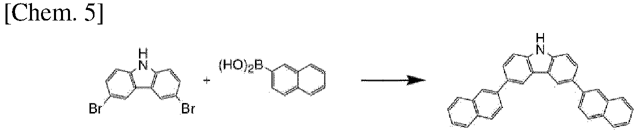

Then, 12.0 g of 3,6-di-naphthalene-2-yl-9H-carbazole, 9.8 g of 3-(4-bromophenyl)-quinoline, 0.3 g of copper(I) iodide, 2.5 g of 1,2-dimethylethylenediamine, and 12.1 g of tripotassium phosphate were added to the reaction vessel, and the mixture was refluxed and stirred in toluene solvent overnight. After the mixture was allowed to cool, the solid was extracted through filtration to obtain a crude product. The obtained crude product was purified through crystallization using monochlorobenzene/acetone mixed solvent to obtain 11.0 g of white powder of 3,6-di-naphthalene-2-yl-9-(4-quinoline-3-yl-phenyl)-9H-carbazole (Compound 1-1) (yield: 61.8%).

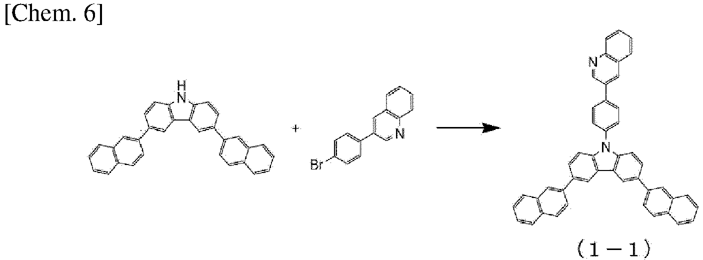

The structure of the obtained white powder was identified using NMR.

In ¹H-NMR (CDCl₃), the following signals of 30 hydrogens were detected, and thus it was seen that the obtained material was Compound (1-1).

δ (ppm) = 9.33 (1H), 8.60 (2H), 8.46 (1H), 8.21 (1H), 8.20 (2H), 8.04-7.81 (15H), 7.79 (1H), 7.64 (3H), 7.51 (4H).

### Example 2

### <Synthesis of 3,6-di-naphthalene-2-yl-9-(4-dibenzothiophene-2-yl-phenyl)-9H-carbazole (Compound 1-11)>

In this example, 10.0 g of 3,6-di-naphthalene-2-yl-9H-carbazole, 9.7 g of 2-(4-bromophenyl)-dibenzothiophene, 0.2 g of copper(I) iodide, 2.1 g of 1,2-dimethylethylenediamine, and 10.1 g of tripotassium phosphate were added to a reaction vessel, and the mixture was refluxed and stirred in toluene solvent overnight. After the mixture was allowed to cool, the solid was extracted through filtration to obtain a crude product. The obtained crude product was purified through crystallization using monochlorobenzene/acetone mixed solvent to obtain 11.7 g of white powder of 3,6-di-naphthalene-2-yl-9-(4-dibenzothiophene-2-yl-phenyl)-9H-carbazole (Compound 1-11) (yield: 72.4%).

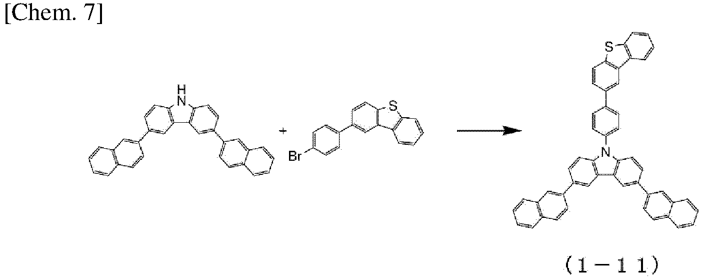

The structure of the obtained white powder was identified using NMR.

In ¹H-NMR (CDCl₃), the following signals of 31 hydrogens were detected, and thus it was seen that the obtained material was Compound (1-11).

δ (ppm) = 8.60 (2H), 8.49 (1H), 8.29 (1H), 8.20 (2H), 8.02-7.81 (15H), 7.79 (2H), 7.64 (2H), 7.53-7.46 (6H).

### Example 3

### <Synthesis of 3,6-di-naphthalene-2-yl-9-(4-benzoxazole-2-yl-phenyl)-9H-carbazole (Compound 1-16)>

In this example, 10.0 g of 3,6-di-naphthalene-2-yl-9H-carbazole, 7.8 g of 2-(4-bromophenyl)-benzoxazole, 0.2 g of copper(I) iodide, and 2.1 g of 1,2-dimethylethylenediamine, and 10.1 g of tripotassium phosphate were added to a reaction vessel, and the mixture was refluxed and stirred in toluene solvent overnight. After the mixture was allowed to cool, the solid was extracted through filtration to obtain a crude product. The obtained crude product was purified through recrystallization using o-dichlorobenzene solvent to obtain 11.2 g of white powder of 3,6-di-naphthalene-2-yl-9-(4-benzoxazole-2-yl-phenyl)-9H-carbazole (Compound 1-16) (yield: 76.7%).

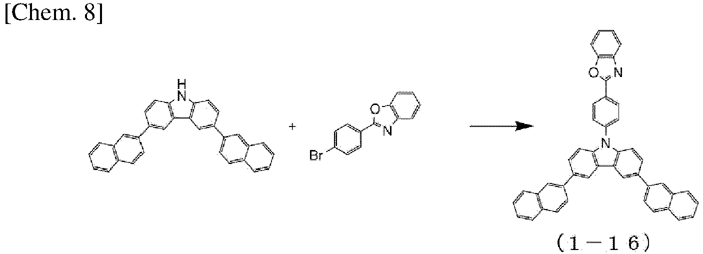

The structure of the obtained white powder was identified using NMR.

In ¹H-NMR (DMSO-d₆), the following signals of 28 hydrogens were detected, and thus it was seen that the obtained material was Compound (1-16).

δ (ppm) = 9.01 (2H), 8.57 (2H), 8.42 (2H), 8.12-8.01 (10H), 8.00 (2H), 7.90 (2H), 7.74 (2H), 7.62-7.45 (6H).

### Example 4

### <Synthesis of 3,6-di-naphthalene-2-yl-9-(4-benzothiazole-2-yl-phenyl)-9H-carbazole (Compound 1-17)>

In this example, 10.0 g of 3,6-di-naphthalene-2-yl-9H-carbazole, 8.3 g of 2-(4-bromophenyl)-benzothiazole, 0.2 g of copper(I) iodide, 2.1 g of 1,2-dimethylethylenediamine, and 10.1 g of tripotassium phosphate were added to a reaction vessel, and the mixture was refluxed and stirred in toluene solvent overnight. After the mixture was allowed to cool, the solid was extracted through filtration to obtain a crude product. The obtained crude product was purified through recrystallization using monochlorobenzene solvent to obtain 6.3 g of pale yellow powder of 3,6-di-naphthalene-2-yl-9-(4-benzothiazole-2-yl-phenyl)-9H-carbazole (Compound 1-17) (yield: 42.0%).

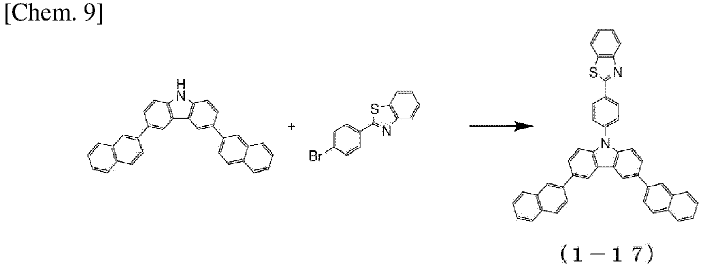

The structure of the obtained pale yellow powder was identified using NMR.

In ¹H-NMR (DMSO-d₆), the following signals of 28 hydrogens were detected, and thus it was seen that the obtained material was Compound (1-17).

δ (ppm) = 9.00 (2H), 8.46 (2H), 8.41 (2H), 8.24 (1H), 8.16 (1H), 8.13-7.95 (12H), 7.72 (2H), 7.65-7.50 (6H).

### Example 5

### <Synthesis of 9-(4-naphthalene-2-yl-phenyl)-3,6-di-quinoline-3-yl-9H-carbazole (Compound 1-27)>

In this example, 121.0 g of 3,6-dibromo-9H-carbazole, 154.6 g of 3-quinolineboronic acid, 17.2 g of tetrakis(triphenylphosphine)palladium(0), and 154.4 g of potassium carbonate were added to a reaction vessel, and the mixture was refluxed and stirred in toluene/EtOH/H₂O mixed solvent overnight. After the mixture was allowed to cool, methanol/H₂O was added to the system, and the solid was extracted through filtration to obtain a crude product. The obtained crude product was purified through crystallization using monochlorobenzene/acetone mixed solvent to obtain 62.1 g of 3,6-di-quinoline-3-yl-9H-carbazole (yield: 39.6%).

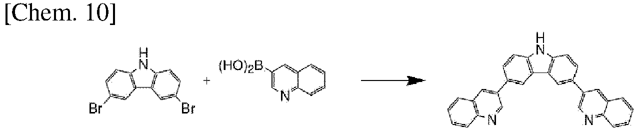

Then, 7.5 g of 3,6-di-quinoline-3-yl-9H-carbazole, 5.5 g of 2-(4-bromophenyl)naphthalene, 0.2 g of bis(tri-t-butylphosphine)palladium(0), and 3.4 g of sodium t-butoxy were added to the reaction vessel, and the mixture was refluxed and stirred in xylene solvent overnight. After the mixture was allowed to cool, methanol was added to the system, and the solid was extracted through filtration to obtain a crude product. The obtained crude product was purified through crystallization using monochlorobenzene/acetone mixed solvent to obtain 3.0 g of white powder of 9-(4-naphthalene-2-yl-phenyl)-3,6-di-quinoline-3-yl-9H-carbazole (Compound 1-27) (yield: 27.0%).

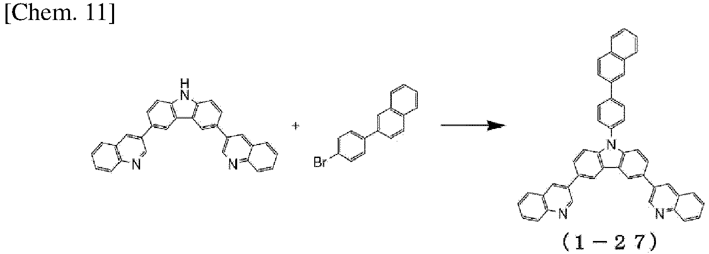

The structure of the obtained white powder was identified using NMR.

In ¹H-NMR (DMSO-d₆), the following signals of 29 hydrogens were detected, and thus it was seen that the obtained material was Compound (1-27).

δ (ppm) = 9.50 (2H), 9.09 (2H), 8.80 (2H), 8.43 (1H), 8.21 (2H), 8.14-8.06 (8H), 8.03 (2H), 7.91 (2H), 7.80 (2H), 7.69 (4H), 7.60 (2H).

### Example 6

### <Synthesis of 9-(4-phenanthrene-9-yl-phenyl)-3,6-di-quinoline-3-yl-9H-carbazole (Compound 1-28)>

In this example, 20.0 g of 3,6-di-quinoline-3-yl-9H-carbazole, 17.4 g of 9-(4-bromophenyl)-phenanthrene, 0.5 g of bis(tri-t-butylphosphine)palladium(0), and 9.2 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 15.0 g of pale yellow powder of 9-(4-phenanthrene-9-yl-phenyl)-3,6-di-quinoline-3-yl-9H-carbazole (Compound 1-28) (yield: 46.9%).

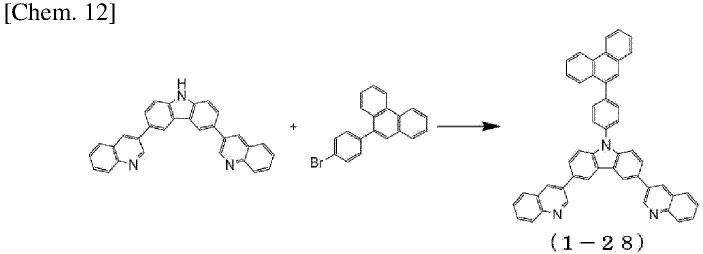

The structure of the obtained pale yellow powder was identified using NMR.

In ¹H-NMR (DMSO-d₆), the following signals of 31 hydrogens were detected, and thus it was seen that the obtained material was Compound (1-28).

δ (ppm) = 9.51 (2H), 9.11 (2H), 9.03 (1H), 8.95 (1H), 8.82 (2H), 8.18-8.06 (8H), 8.02 (1H), 7.96 (4H), 7.86-7.73 (8H), 7.70 (2H).

### Example 7

### <Synthesis of 9-(4-dibenzothiophene-2-yl-phenyl)-3,6-di-quinoline-3-yl-9H-carbazole (Compound 1-34)>

In this example, 15.0 g of 3,6-di-quinoline-3-yl-9H-carbazole, 13.3 g of 2-(4-bromophenyl)-dibenzothiophene, 0.4 g of bis(tri-t-butylphosphine)palladium(0), and 6.9 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 9.1 g of pale yellow powder of 9-(4-dibenzothiophene-2-yl-phenyl)-3,6-di-quinoline-3-yl-9H-carbazole (Compound 1-34) (yield: 37.6%).

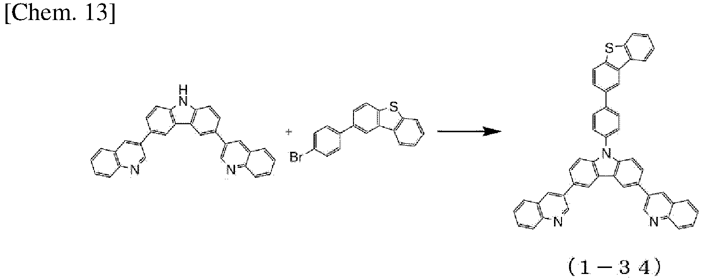

The structure of the obtained pale yellow powder was identified using NMR.

In ¹H-NMR (DMSO-d₆), the following signals of 29 hydrogens were detected, and thus it was seen that the obtained material was Compound (1-34).

δ (ppm) = 9.50 (2H), 9.10 (2H), 8.90 (1H), 8.81 (2H), 8.61 (1H), 8.25 (2H), 8.22 (1H), 8.17-8.06 (7H), 8.03 (1H), 7.92 (2H), 7.90 (2H), 7.70 (4H), 7.59 (2H).

### Example 8

### <Synthesis of 9-(4-dibenzofuran-3-yl-phenyl)-3,6-di-quinoline-3-yl-9H-carbazole (Compound 1-35)>

In this example, 10.0 g of 3,6-di-quinoline-3-yl-9H-carbazole, 8.4 g of 3-(4-bromophenyl)-dibenzofuran, 0.4 g of bis(tri-t-butylphosphine)palladium(0), and 3.4 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 6.0 g of white powder of 9-(4-dibenzofuran-3-yl-phenyl)-3,6-di-quinoline-3-yl-9H-carbazole (Compound 1-35) (yield: 38.2%).

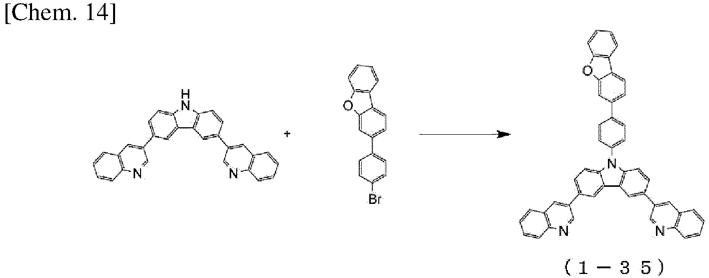

The structure of the obtained white powder was identified using NMR.

In ¹H-NMR (CDCl₃), the following signals of 29 hydrogens were detected, and thus it was seen that the obtained material was Compound (1-35).

δ (ppm) = 9.37 (2H), 8.61 (2H), 8.47 (2H), 8.10 (2H), 8.02 (1H), 8.01-7.93 (6H), 8.86 (2H), 7.78-7.72 (5H), 7.69 (2H), 7.62 (3H), 7.51 (1H), 5.40 (1H).

### Example 9

### <Synthesis of 9-(4-benzoxazole-2-yl-phenyl)-3,6-di-quinoline-3-yl-9H-carbazole (Compound 1-37)>

In this example, 20.0 g of 3,6-di-quinoline-3-yl-9H-carbazole, 14.3 g of 2-(4-bromophenyl)-benzoxazole, 0.5 g of bis(tri-t-butylphosphine)palladium(0), and 9.2 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 15.0 g of white powder of 9-(4-benzoxazole-2-yl-phenyl)-3,6-di-quinoline-3-yl-9H-carbazole (Compound 1-37) (yield: 51.5%).

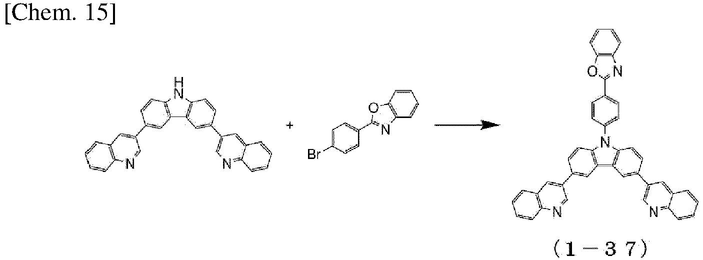

The structure of the obtained white powder was identified using NMR.

In ¹H-NMR (DMSO-d₆), the following signals of 26 hydrogens were detected, and thus it was seen that the obtained material was Compound (1-37).

δ (ppm) = 9.50 (2H), 9.10 (2H), 8.82 (2H), 8.58 (2H), 8.16-8.09 (6H), 8.07 (2H), 7.91 (2H), 7.79 (4H), 7.70 (2H), 7.50 (2H).

### Example 10

### <Synthesis of 9-(4-benzothiazole-2-yl-phenyl)-3,6-di-quinoline-3-yl-9H-carbazole (Compound 1-38)>

In this example, 7.5 g of 3,6-di-quinoline-3-yl-9H-carbazole, 5.7 g of 2-(4-bromophenyl)-benzothiazole, 0.2 g of bis(tri-t-butylphosphine)palladium(0), and 3.4 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through crystallization using monochlorobenzene/acetone mixed solvent to obtain 3.2 g of pale yellow powder of 9-(4-benzothiazole-2-yl-phenyl)-3,6-di-quinoline-3-yl-9H-carbazole (Compound 1-38) (yield: 28.5%).

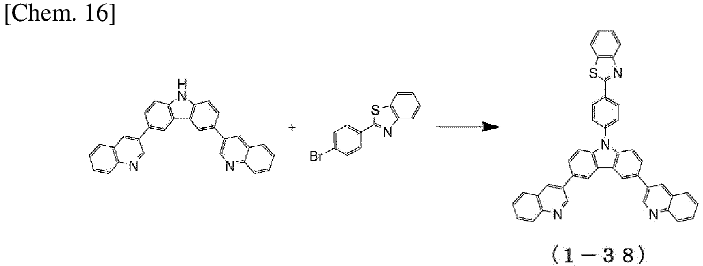

The structure of the obtained pale yellow powder was identified using NMR.

In ¹H-NMR (DMSO-d₆), the following signals of 26 hydrogens were detected, and thus it was seen that the obtained material was Compound (1-38).

δ (ppm) = 9.49 (2H), 9.09 (2H), 8.81 (2H), 8.47 (2H), 8.24 (1H), 8.16 (1H), 8.14-8.05 (6H), 8.00 (2H), 7.78 (4H), 7.69 (2H), 7.62 (1H), 7.54 (1H).

### Example 11

### <Synthesis of 9-(4-benzofuran-2-yl-phenyl)-3,6-di-quinoline-3-yl-9H-carbazole (Compound 1-39)>

In this example, 11.0 g of 3,6-di-quinoline-3-yl-9H-carbazole, 7.8 g of 2-(4-bromophenyl)-benzofuran, 0.4 g of bis(tri-t-butylphosphine)palladium(0), and 3.8 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 5.3 g of white powder of 9-(4-benzofuran-2-yl-phenyl)-3,6-di-quinoline-3-yl-9H-carbazole (Compound 1-39) (yield: 33.2%).

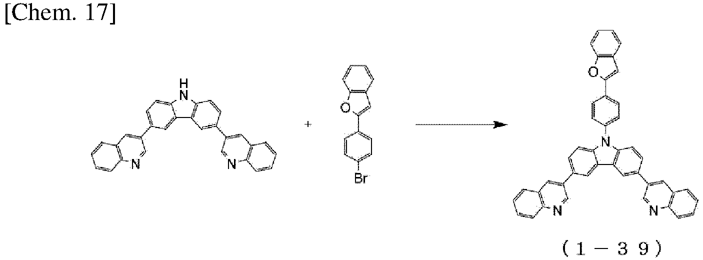

The structure of the obtained white powder was identified using NMR.

In ¹H-NMR (DMSO-d₆), the following signals of 27 hydrogens were detected, and thus it was seen that the obtained material was Compound (1-39).

δ (ppm) = 9.49 (2H), 9.09 (2H), 8.81 (2H), 8.30 (2H), 8.13-8.08 (6H), 7.93 (2H), 7.82-7.66 (9H), 7.42-7.32 (2H).

### Example 12

### <Synthesis of 9-(4-benzothiophene-2-yl-phenyl)-3,6-di-quinoline-3-yl-9H-carbazole (Compound 1-40)>

In this example, 14.7 g of 3,6-di-quinoline-3-yl-9H-carbazole, 11.1 g of 2-(4-bromophenyl)-benzothiophene, 0.5 g of bis(tri-t-butylphosphine)palladium(0), and 5.0 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 11.0 g of white powder of 9-(4-benzothiophene-2-yl-phenyl)-3,6-di-quinoline-3-yl-9H-carbazole (Compound 1-40) (yield: 50.0%).

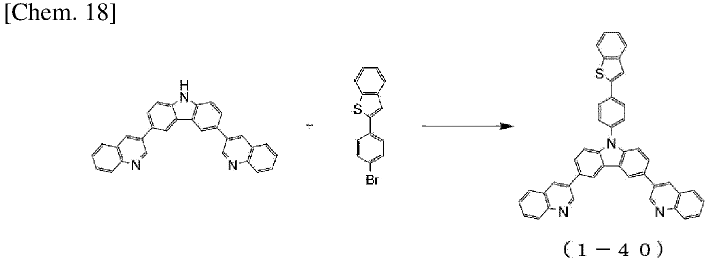

The structure of the obtained white powder was identified using NMR.

In ¹H-NMR (DMSO-d₆), the following signals of 27 hydrogens were detected, and thus it was seen that the obtained material was Compound (1-40).

δ (ppm) = 9.50 (2H), 9.09 (2H), 8.81 (2H), 8.18-8.06 (10H), 7.95-7.89 (3H), 7.81-7.78 (2H), 7.72-7.67 (4H), 7.48-7.41 (2H).

### Example 13

### <Synthesis of 9-naphthalene-2-yl-3-(3,5-di-quinoline-3-yl-phenyl)-9H-carbazole (Compound 1-64)>

In this example, 5.0 g of 3-bromo-9-naphthalene-2-yl-9H-carbazole, 6.5 g of 4,4,5,5-tetramethyl-[1,3,2]dioxaborolane-2-yl-3,5-di-quinoline-3-yl-benzene, 0.3 g of tetrakis(triphenylphosphine)palladium(0), and 3.7 g of potassium carbonate were added to a reaction vessel, and the mixture was refluxed and stirred in toluene/EtOH/H₂O mixed solvent overnight. After the mixture was allowed to cool, methanol/H₂O was added to the system, and the solid was extracted through filtration to obtain a crude product. The obtained crude product was purified through crystallization using toluene/acetone mixed solvent to obtain 4.1 g of white powder of 9-naphthalene-2-yl-3-(3,5-di-quinoline-3-yl-phenyl)-9H-carbazole (Compound 1-64) (yield: 48.9%).

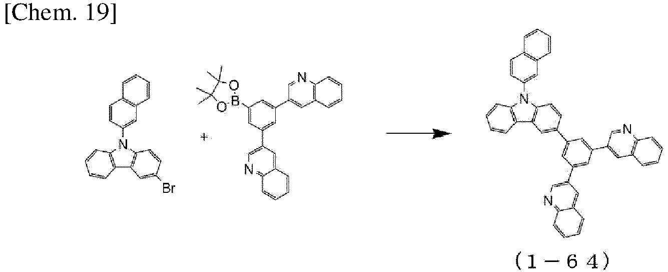

The structure of the obtained white powder was identified using NMR.

In ¹H-NMR (CDCl₃), the following signals of 29 hydrogens were detected, and thus it was seen that the obtained material was Compound (1-64).

δ (ppm) = 9.37 (2H), 8.54 (1H), 8.50 (2H), 8.23 (1H), 8.20 (2H), 8.11 (4H), 8.03-7.91 (5H), 7.80 (2H), 7.76 (1H), 7.71 (1H), 7.66-7.56 (5H), 7.48 (2H), 7.36 (1H).

### Example 14

### <Synthesis of 9-phenyl-3-(3,5-di-quinoline-3-yl-biphenyl-4'-yl)-9H-carbazole (Compound 1-66)>

In this example, 5.0 g of 3-(4-bromophenyl)-9-phenyl-9H-carbazole, 6.0 g of 4,4,5,5-tetramethyl-[1,3,2]dioxaborolane-2-yl-3,5-di-quinoline-3-yl-benzene, 0.3 g of tetrakis(triphenylphosphine)palladium(0), and 3.5 g of potassium carbonate were added to a reaction vessel, and the mixture was refluxed and stirred in toluene/EtOH/H₂O mixed solvent overnight. After the mixture was allowed to cool, methanol/H₂O was added to the system, and the solid was extracted through filtration to obtain a crude product. The obtained crude product was purified through crystallization using toluene/acetone mixed solvent to obtain 3.9 g of white powder of 9-phenyl-3-(3,5-di-quinoline-3-yl-biphenyl-4'-yl)-9H-carbazole (Compound 1-66) (yield: 47.8%).

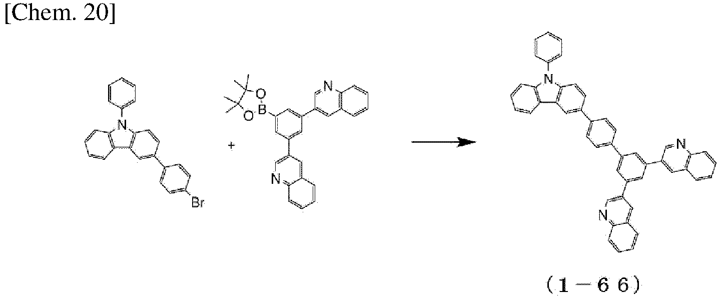

The structure of the obtained white powder was identified using NMR.

In ¹H-NMR (CDCl₃), the following signals of 31 hydrogens were detected, and thus it was seen that the obtained material was Compound (1-66).

δ (ppm) = 9.35 (2H), 8.48 (2H), 8.44 (1H), 8.23 (1H), 8.20 (2H), 8.06 (2H), 8.02 (1H), 7.96 (2H), 7.89 (4H), 7.78 (2H), 7.74 (1H), 7.67-7.58 (6H), 7.50 (2H), 7.44 (2H), 7.33 (1H).

### Example 15

### <Synthesis of 3-(4-benzofuran-2-yl-phenyl)-9-(4-benzoxazole-2-yl-phenyl)-9H-carbazole (Compound 1-70)>

In this example, 5.0 g of 3-(4-benzofuran-2-yl-phenyl)-9H-carbazole, 4.6 g of 2-(4-bromophenyl)-benzoxazole, 0.3 g of tris(dibenzylideneacetone)dipalladium(0), 0.3 g of tri-t-butylphosphine, and 2.0 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using 1,2-dichlorobenzene solvent to obtain 6.9 g of white powder of 3-(4-benzofuran-2-yl-phenyl)-9-(4-benzoxazole-2-yl-phenyl)-9H-carbazole (Compound 1-70) (yield: 89.7%).

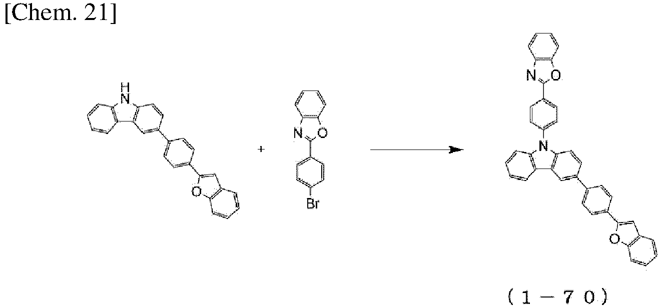

The structure of the obtained white powder was identified using NMR.

In ¹H-NMR (DMSO-d₆), the following signals of 24 hydrogens were detected, and thus it was seen that the obtained material was Compound (1-70).

δ (ppm) = 8.75 (1H), 8.53 (2H), 8.44 (1H), 8.08 (2H), 8.01-7.96 (4H), 7.93-7.87 (3H), 7.71-7.66 (3H), 7.61 (1H), 7.55-7.46 (4H), 7.42-7.28 (3H).

### Example 16

### <Synthesis of 9-(4'-naphthalene-2-yl-[1,1'] biphenyl-4-yl)-3,6-di-quinoline-3-yl-9H-carbazole (Compound 1-85)>

In this example, 10.0 g of 3,6-di-quinoline-3-yl-9H-carbazole, 9.2 g of 2-(4'-bromo-[1,1'] biphenyl-4-yl)-naphthalene, 0.4 g of bis(tri-t-butylphosphine)palladium(0), and 3.4 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 6.8 g of white powder of 9-(4'-naphthalene-2-yl-[1,1'] biphenyl-4-yl)-3,6-di-quinoline-3-yl-9H-carbazole (Compound 1-85) (yield: 40.7%).

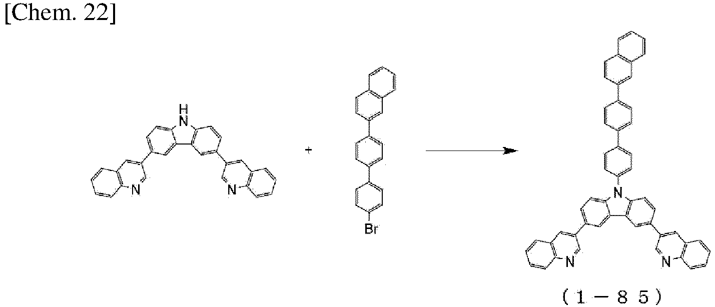

The structure of the obtained white powder was identified using NMR.

In ¹H-NMR (CDCl₃), the following signals of 33 hydrogens were detected, and thus it was seen that the obtained material was Compound (1-85).

δ (ppm) = 9.38 (2H), 8.61 (2H), 8.47 (2H), 8.17 (3H), 7.99-7.84 (14H), 7.77-7.72 (4H), 7.69 (2H), 7.61 (2H), 7.57-7.50 (2H).

### Example 17

### <Synthesis of 9-(4-phenanthrene-2-yl-phenyl)-3,6-di-quinoline-3-yl-9H-carbazole (Compound 1-86)>

In this example, 10.0 g of 3,6-di-quinoline-3-yl-9H-carbazole, 8.7 g of 2-(4-bromophenyl)-phenanthrene, 0.4 g of tris(dibenzylideneacetone)dipalladium(0), 0.2 g of tri-t-butylphosphine, and 4.6 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 8.4 g of white powder of 9-(4-phenanthrene-2-yl-phenyl)-3,6-di-quinoline-3-yl-9H-carbazole (Compound 1-86) (yield: 52.2%).

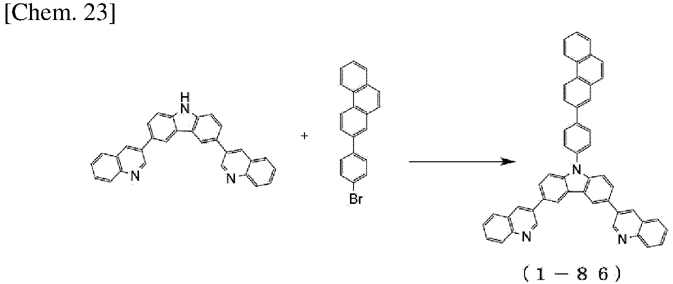

The structure of the obtained white powder was identified using NMR.

In ¹H-NMR (CDCl₃), the following signals of 31 hydrogens were detected, and thus it was seen that the obtained material was Compound (1-86).

δ (ppm) = 9.38 (2H), 8.84 (1H), 8.76 (1H), 8.61 (2H), 8.47 (2H), 8.25 (1H), 8.19 (2H), 8.09-8.03 (3H), 7.95 (3H), 7.89-7.84 (4H), 7.80 (2H), 7.76-7.60 (8H).

### Example 18

### <Synthesis of 9-(7-benzothiophene-2-yl-naphthalene-2-yl)-3,6-di-quinoline-3-yl-9H-carbazole (Compound 1-91)>

In this example, 10.0 g of 3,6-di-quinoline-3-yl-9H-carbazole, 8.9 g of 2-(7-bromo-naphthalene-2-yl)-benzothiophene, 0.4 g of bis(tri-t-butylphosphine)palladium(0), and 3.4 g of sodium t-butoxy were added to a reaction vessel, and the mixture was refluxed and stirred in xylene solvent overnight. After the mixture was allowed to cool, methanol was added to the system, and the solid was extracted through filtration to obtain a crude product. The obtained crude product was purified through recrystallization using monochlorobenzene solvent to obtain 8.4 g of white powder of 9-(7-benzothiophene-2-yl-naphthalene-2-yl)-3,6-di-quinoline-3-yl-9H-carbazole (Compound 1-91) (yield: 52.2%).

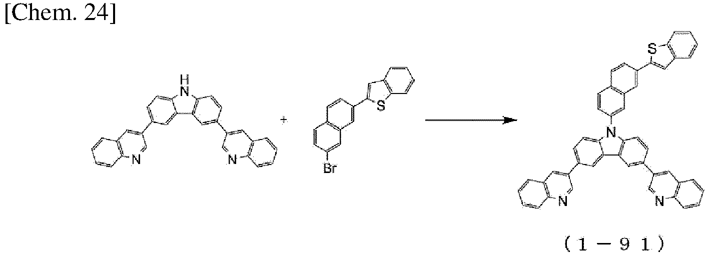

The structure of the obtained white powder was identified using NMR.

In ¹H-NMR (CDCl₃), the following signals of 29 hydrogens were detected, and thus it was seen that the obtained material was Compound (1-91).

δ (ppm) = 9.38 (2H), 8.62 (2H), 8.47 (2H), 8.26 (1H), 8.20-8.14 (4H), 8.08-8.00 (2H), 7.95 (2H), 7.89-7.84 (4H), 7.76-7.72 (4H), 7.68 (2H), 7.61 (2H), 7.38 (2H).

### Example 19

### <Synthesis of 9-(4-(2-phenyl-benzoxazole-6-yl)-phenyl)-3,6-di-quinoline-3-yl-9H-carbazole (Compound 1-92)>

In this example, 5.0 g of 3,6-di-quinoline-3-yl-9H-carbazole, 4.6 g of 6-(4-chlorophenyl)-2-phenyl-benzoxazole, 0.2 g of tris(dibenzylideneacetone)dipalladium(0), 0.1 g of tri-t-butylphosphine, and 2.3 g of sodium t-butoxy were added to a reaction vessel, and the mixture was refluxed and stirred in toluene solvent overnight. After the mixture was allowed to cool, methanol was added to the system, and the solid was extracted through filtration to obtain a crude product. The obtained crude product was purified through crystallization using monochlorobenzene/acetone mixed solvent to obtain 4.1 g of pale yellow powder of 9-(4-(2-phenyl-benzoxazole-6-yl)-phenyl)-3,6-di-quinoline-3-yl-9H-carbazole (Compound 1-92) (yield: 50.0%).

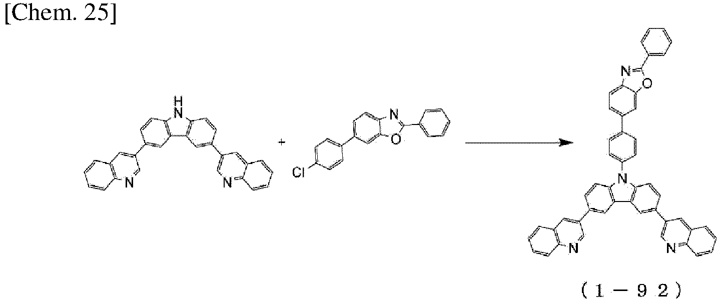

The structure of the obtained pale yellow powder was identified using NMR.

In ¹H-NMR (CDCl₃), the following signals of 30 hydrogens were detected, and thus it was seen that the obtained material was Compound (1-92).

δ (ppm) = 9.36 (2H), 8.58 (2H), 8.44 (2H), 8.31 (2H), 8.18 (2H), 7.94-7.88 (6H), 7.83 (2H), 7.73 (5H), 7.66 (2H), 7.62-7.56 (5H).

### Example 20

The melting point and the glass transition point of each of the compounds obtained in Examples 1 to 19 above were measured using a high-sensitivity differential scanning calorimeter (DSC3100SA manufactured by Bruker AXS K.K.). Table 1 shows the results.

**Table 1**

| | Example compound | Melting point | Glass transition point |
|---|---|---|---|
| Ex. 1 | Compound (1-1) | 265 °C | 116°C |
| Ex. 2 | Compound (1-11) | 271°C | 125°C |
| Ex. 3 | Compound (1-16) | 299°C | - |
| Ex. 4 | Compound (1-17) | 295 °C | 117°C |
| Ex. 5 | Compound (1-27) | 288°C | - |
| Ex. 6 | Compound (1-28) | 303°C | 147°C |
| Ex. 7 | Compound (1-34) | 271°C | 140°C |
| Ex. 8 | Compound (1-35) | - | 133°C |
| Ex. 9 | Compound (1-37) | 309°C | - |
| Ex. 10 | Compound (1-38) | 313°C | - |
| Ex. 11 | Compound (1-39) | 308°C | - |
| Ex. 12 | Compound (1-40) | 315°C | - |
| Ex. 13 | Compound (1-64) | - | 125°C |
| Ex. 14 | Compound (1-66) | - | 124°C |
| Ex. 15 | Compound (1-70) | 293°C | 106°C |
| Ex. 16 | Compound (1-85) | 280°C | 134°C |
| Ex. 17 | Compound (1-86) | 294°C | 143°C |
| Ex. 18 | Compound (1-91) | 282°C | 148°C |
| Ex. 19 | Compound (1-92) | 287°C | 137°C |

It is seen from the results that most of the compounds obtained in Examples 1 to 19 had a glass transition point of 100°C or higher, which indicates that these compounds are stable in a thin film state.

### Example 21

A vapor-deposited film with a film thickness of 80 nm was formed on a silicon substrate using each of the compounds represented by the general formula (1) or (2) obtained in Examples 1 to 19 above, and a refractive index n and an extinction coefficient k at wavelengths of 450 and 750 nm were measured using a spectroscopic measurement device (F10-RT-UV manufactured by Filmetrics). For comparison, the measurement was also performed on Alq₃ and Comparative Compound (2-1) having the structural formula below (see Patent Literature 4, for example). Table 2 collectively shows the measurement results.

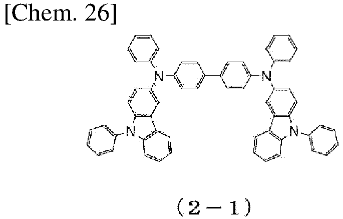

**Table 2**

| | | Refractive index: n (λ: 450 nm) | Extinction coefficient: k (λ: 450 nm) | Refractive index: n (λ: 750 nm) | Extinction coefficient: k (λ: 750 nm) |
|---|---|---|---|---|---|
| Ex. 1 | Compound (1-1) | 2.11 | 0 | 1.92 | 0 |
| Ex. 2 | Compound (1-11) | 2.11 | 0 | 1.93 | 0 |
| Ex. 3 | Compound (1-16) | 2.12 | 0 | 1.91 | 0 |
| Ex. 4 | Compound (1-17) | 2.15 | 0 | 1.93 | 0 |
| Ex. 5 | Compound (1-27) | 2.15 | 0 | 1.93 | 0 |
| Ex. 6 | Compound (1-28) | 2.15 | 0 | 1.94 | 0 |
| Ex. 7 | Compound (1-34) | 2.16 | 0 | 1.95 | 0 |
| Ex. 8 | Compound (1-35) | 2.16 | 0 | 1.94 | 0 |
| Ex. 9 | Compound (1-37) | 2.18 | 0 | 1.93 | 0 |
| Ex. 10 | Compound (1-38) | 2.20 | 0 | 1.94 | 0 |
| Ex. 11 | Compound (1-39) | 2.17 | 0 | 1.93 | 0 |
| Ex. 12 | Compound (1-40) | 2.16 | 0 | 1.93 | 0 |
| Ex. 13 | Compound (1-64) | 2.05 | 0 | 1.90 | 0 |
| Ex. 14 | Compound (1-66) | 2.06 | 0 | 1.89 | 0 |
| Ex. 15 | Compound (1-70) | 2.14 | 0 | 1.88 | 0 |
| Ex. 16 | Compound (1-85) | 2.16 | 0 | 1.94 | 0 |
| Ex. 17 | Compound (1-86) | 2.17 | 0 | 1.94 | 0 |
| Ex. 18 | Compound (1-91) | 2.16 | 0 | 1.94 | 0 |
| Ex. 19 | Compound (1-92) | 2.17 | 0 | 1.93 | 0 |
| Com.Ex. 1 | Alq3 | 1.88 | 0 | 1.73 | 0 |
| Com.Ex. 2 | Compound (2-1) | 1.84 | 0 | 1.77 | 0 |

As shown in Table 2, the refractive indices of the compounds of the present invention were similar to or higher than those of Alq₃ and Comparative Compound (2-1) in a wavelength range of 450 to 750 nm, that is, an improvement in light extraction efficiency can be expected in an organic EL element in which any of the compounds of the present invention is used as a constituent material of the capping layer.

### Example 22

An organic EL element was produced using Compound (1-1) obtained in Example 1 above, and the properties thereof were evaluated.

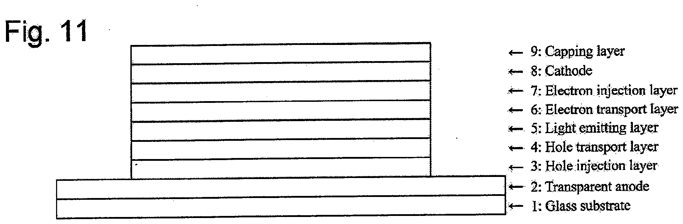

As shown in Fig. 11, an organic EL element was prepared by forming a reflecting ITO electrode serving as a metal anode 2 on a glass substrate 1 in advance, and furthermore, vapor-depositing a hole injection layer 3, a hole transport layer 4, a light emitting layer 5, an electron transport layer 6, an electron injection layer 7, a cathode 8, and a capping layer 9 in this order on the ITO electrode.

Specifically, a glass substrate 1 on which an ITO film with a thickness of 50 nm, a reflecting film of silver alloy with a film thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 2 minutes. Then, the glass substrate with ITO was attached inside a vacuum vapor deposition machine, and the pressure was reduced to 0.001 Pa or less.

Subsequently, an electron acceptor (Acceptor-1) having the structural formula below and Compound (3-1) having the structural formula below were vapor-deposited so as to cover the transparent anode 2 through binary vapor deposition at such vapor deposition rates that the ratio of the vapor deposition rate of (Acceptor-1) to the vapor deposition rate of Compound (3-1) was 3:97, and a film with a thickness of 10 nm was thus formed as a hole injection layer 3.

A film of Compound (3-1) having the structural formula below was formed on the hole injection layer 3 as a hole transport layer 4 with a film thickness of 140 nm.

Compound (3-2) having the structural formula below and Compound (3-3) having the structural formula below were vapor-deposited on the hole transport layer 4 through binary vapor deposition at such vapor deposition rates that the ratio of the vapor deposition rate of (3-2) to the vapor deposition rate of (3-3) was 5:95, and a film with a thickness of 20 nm was thus formed as a light emitting layer 5.

Compound (3-4) having the structural formula below and Compound (3-5) having the structural formula below were vapor-deposited on the light emitting layer 5 through binary vapor deposition at such vapor deposition rates that the ratio of the vapor deposition rate of (3-4) to the vapor deposition rate of (3-5) was 50:50, and a film with a thickness of 30 nm was thus formed as an electron transport layer 6.

A film of lithium fluoride was formed on the electron transport layer 6, as an electron injection layer 7 with a film thickness of 1 nm.

A film of magnesium-silver alloy was formed on the electron injection layer 7, as a cathode 8 with a film thickness of 12 nm.

Lastly, a film of Compound (1-1) of Example 1 was formed as a capping layer 9 with a film thickness of 60 nm.

Table 3 collectively shows the measurement results of light emission properties that were obtained when a DC voltage was applied to the produced organic EL element in the atmosphere at normal temperature.

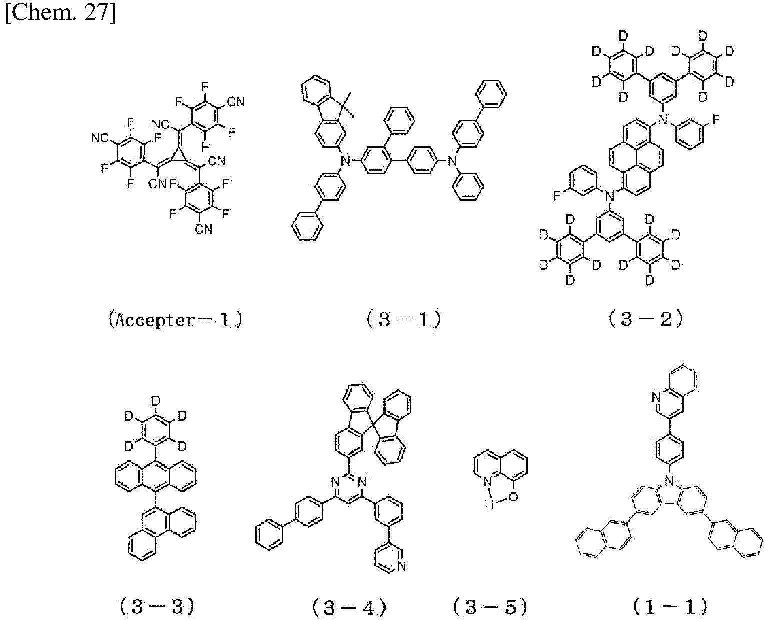

### Examples 23 to 40

An organic EL element was prepared under similar conditions to those of Example 22, except that the compounds of Examples 2 to 19 were used instead of Compound (1-1) of Example 1 to form a film with a thickness of 60 nm as the capping layer 9. Table 3 collectively shows the measurement results of light emission properties that were obtained when a DC voltage was applied to the produced organic EL elements in the atmosphere at normal temperature.

### Comparative Example 1

For the sake of comparison, an organic EL element was prepared under similar conditions to those of Example 22, except that Alq₃ was used instead of Compound (1-1) of Example 1 to form a film with a thickness of 60 nm as the capping layer 9. Table 3 collectively shows the measurement results of light emission properties that were obtained when a DC voltage was applied to the produced organic EL element in the atmosphere at normal temperature.

### Comparative Example 2

For the sake of comparison, an organic EL element was prepared under similar conditions to those of Example 22, except that Compound (2-1) was used instead of Compound (1-1) of Example 1 to form a film with a thickness of 60 nm as the capping layer 9. Table 3 collectively shows the measurement results of light emission properties that were obtained when a DC voltage was applied to the produced organic EL element in the atmosphere at normal temperature.

The voltage, the luminance, the light emission efficiency, and the power efficiency in Table 3 are values at a current density of 10 mA/cm², and the element lifespan was defined as follows: when constant current driving was performed at a current density of 10 mA/cm² with the initial luminance being set to 100%, the time taken for the luminance to decay to 95% was measured as the element lifespan.

**Table 3**

| | Capping layer | Voltage [V] (@10 mA/cm²) | Luminance [cd/m²] (@10 mA/cm²) | Light emission efficiency [cd/A] (@10 mA/cm²) | Power efficiency [lm/W] (@10 mA/cm²) | Element lifespan to decay of 95% |
|---|---|---|---|---|---|---|
| Ex. 22 | Compound (1-1) | 3.66 | 812 | 8.12 | 6.97 | 157 hours |
| Ex. 23 | Compound (1-11) | 3.67 | 810 | 8.10 | 6.94 | 149 hours |
| Ex. 24 | Compound (1-16) | 3.65 | 812 | 8.12 | 6.98 | 152 hours |
| Ex. 25 | Compound (1-17) | 3.66 | 821 | 8.21 | 7.05 | 153 hours |
| Ex. 26 | Compound (1-27) | 3.66 | 817 | 8.18 | 7.01 | 156 hours |
| Ex. 27 | Compound (1-28) | 3.62 | 827 | 8.27 | 7.18 | 143 hours |
| Ex. 28 | Compound (1-34) | 3.63 | 832 | 8.32 | 7.20 | 166 hours |
| Ex. 29 | Compound (1-35) | 3.66 | 824 | 8.24 | 7.07 | 161 hours |
| Ex. 30 | Compound (1-37) | 3.66 | 836 | 8.35 | 7.17 | 159 hours |
| Ex. 31 | Compound (1-38) | 3.63 | 849 | 8.49 | 7.35 | 162 hours |
| Ex. 32 | Compound (1-39) | 3.65 | 820 | 8.20 | 7.07 | 148 hours |
| Ex. 33 | Compound (1-40) | 3.62 | 826 | 8.26 | 7.18 | 160 hours |
| Ex. 34 | Compound (1-64) | 3.64 | 800 | 8.00 | 6.90 | 151 hours |
| Ex. 35 | Compound (1-66) | 3.64 | 797 | 7.96 | 6.88 | 145 hours |
| Ex. 36 | Compound (1-70) | 3.67 | 804 | 8.04 | 6.89 | 163 hours |
| Ex. 37 | Compound (1-85) | 3.64 | 824 | 8.24 | 7.12 | 146 hours |
| Ex. 38 | Compound (1-86) | 3.62 | 821 | 8.21 | 7.14 | 164 hours |
| Ex. 39 | Compound (1-91) | 3.68 | 817 | 8.17 | 6.98 | 150 hours |
| Ex. 40 | Compound (1-92) | 3.67 | 825 | 8.25 | 7.06 | 151 hours |
| Com. Ex. 1 | Alq3 | 3.65 | 714 | 7.14 | 6.15 | 114 hours |
| Com. Ex. 2 | Compound (2-1) | 3.66 | 735 | 7.34 | 6.30 | 133 hours |

As shown in Table 3, while the elements of Comparative Examples 1 and 2 and those of Examples 22 to 40 had substantially similar driving voltages at a current density of 10 mA/cm², all of the elements of the examples were superior to those of the comparative examples in terms of the luminance, the light emission efficiency, the power efficiency, and the element lifespan compared with. This fact means that the compound represented by the general formula (1) or (2) of the present invention is a material that can be favorably used in a capping layer, and can increase the refractive index of the capping layer, thereby being capable of significantly improving the light extraction efficiency of an organic EL element.

### Industrial Applicability

The compound of the present invention has a high refractive index, can significantly improve the light extraction efficiency, and is stable in a thin film state, and thus it is excellent as a compound that is favorably used in an organic EL element. Furthermore, the organic EL element produced using the compound of the present invention has high efficiency. Furthermore, the compound of the present invention that does not have absorption in blue, green, and red wavelength regions is particularly preferably used to provide a clear and bright image with good color purity. Therefore, the present invention is expected to be applied to applications such as home electric appliances and lighting equipment, for example.

### Reference Signs List

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode
- 9: Capping layer

## Claims

1. A compound represented by a general formula (1) or (2) below: 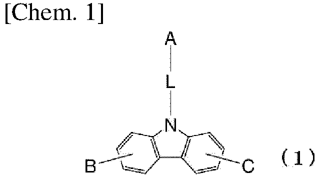 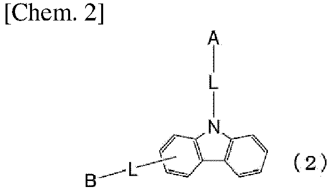
where A represents a substituted or unsubstituted aromatic hydrocarbon group, a substituted or unsubstituted aromatic heterocyclic group, or a substituted or unsubstituted fused polycyclic aromatic group,
B and C are optionally the same or different, and represent an unsubstituted naphthyl group, an unsubstituted quinolyl group, an unsubstituted benzofuranyl group, or an unsubstituted benzothienyl group, and
Each L is optionally the same or different, and represents a single bond, a substituted or unsubstituted divalent aromatic hydrocarbon group, a substituted or unsubstituted divalent aromatic heterocyclic group, or a substituted or unsubstituted divalent fused polycyclic aromatic group.

2. The compound according to claim 1, wherein the compounds represented by the general formulas (1) and (2) mentioned above are respectively the compounds represented by general formulas (3) and (4) below: 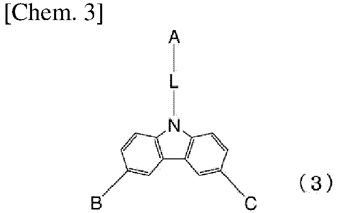 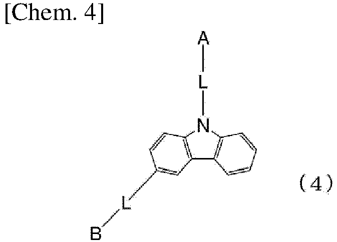 where A, B, C, and L are the same as those defined in the general formulas (1) and (2) above.

3. The compound according to claim 2, wherein B and C in the general formulas (3) and (4) mentioned above are optionally the same or different, and represent a 2-naphthyl group, a 3-quinolyl group, a 2-benzofuranyl group, or a 2-benzothienyl group.

4. The compound according to claim 3, wherein each L in the general formulas (3) and (4) mentioned above is optionally the same or different, and represents a single bond, a substituted or unsubstituted phenylene group, a substituted or unsubstituted biphenylene group, or a substituted or unsubstituted naphthylene group.

5. An organic electroluminescent element at least comprising an anode electrode, a hole transport layer, a light emitting layer, an electron transport layer, a cathode electrode, and a capping layer arranged in this order,
wherein the capping layer contains the compound according to any one of claims 1 to 4.

6. The organic electroluminescent element according to claim 5, wherein the refractive index of the capping layer in a wavelength range of 450 to 750 nm is 1.70 or more.

7. The organic electroluminescent element according to claim 5, wherein the capping layer is stacked layers respectively constituted by two or more types of compounds or a mixed layer containing two or more types of compounds.

8. An electronic element comprising a pair of electrodes and an organic layer held therebetween, wherein the organic layer contains the compound according to any one of claims 1 to 4.

9. An electronic device comprising the electronic element according to claim 8.
